# EUROPEAN PATENT APPLICATION

(11) **EP 1 359 218 A1**
(43) Date of publication of application: **05.11.2003**
(21) Application number: 01961145.8
(22) Date of filing: 29.08.2001
(51) Int. Cl.: C12N 15/11

(54) **RNA SHOWING NEURON SURVIVAL ACTIVITY**

(30) Priority: 09.02.2001 JP 2001033806
(71) Applicant: KABUSHIKI KAISHA FRONTIER, Tokyo-to 169-0051 (JP)
(72) Inventor: MIYATA, Yuhei, Abiko-shi, Chiba-ken 270-1164 (JP); UI-TEI, Kumiko, Tokyo-to 113-0032 (JP); NAGANO, Masatoshi, Kawasaki-shi, Kanagawa-ken 211-0063 (JP); HAMADA, Tsuyoshi, Kobe-shi, Hyogo-ken 651-0087 (JP); TAKAHASHI, Fumitaka, Yokohama-shi, Kanagawa-ken 220-0035 (JP); OHTA, Shigeo, Machida-shi, Tokyo-to 194-0032 (JP)
(74) Representative: Grünecker, Kinkeldey, Stockmair & Schwanhäusser Anwaltssozietät
(86) International application number: JP0107398
(87) International publication number: WO02064778

(57) **Abstract**

The present invention provides an in-vitro-derived component which permits identification of essential components contained in an extracted fraction having physiological activities such as a survival activity on neuron, particularly to spinal cord motoneuron, and is useful for diagnosis, prevention and therapy of various diseases including neurodegenerative diseases. The RNA of the present invention has a base sequence represented by the sequence No.1 of the sequence table, exhibiting a very high survival activity relative to spinal cord motoneuron.

SEQUENCE LISTING

<110> KABUSHIKI KAISHA FRONTIER

<120> RNA exhibiting a neuron survival activity

<130> F13-0133

<160> 1

<210> 1

<211> 299

<212> RNA

<213> Gallus gallus

<400> 1

gccgggcgcg guggcgcacg ccuguagucc cagcuacucg ggaggcugag cccgccggau 60

cgcuugagcc caggaguucu gggcugcagu gcgcuaugcc gagcgggcgu ccgcgcuaag 120

gccggcauca auauggugag ccccggggag ccgaggcaca ccagguugcc uaaggagggg 180

ugaaccggaa caggucggaa acggagcagg ucaaaacucc cgugccgguc aguaacggga 240

ucgcgccugu gaauagccac ugcagcguag ccugggcaac auagcgagac ccugucucc 299

## Description

### Technical Field

The present invention relates to an RNA identified as a component presenting a neuron physiological activity such as a neuron survival activity useful for diagnosis, a prophylaxis or a therapeutic drug of motoneuron diseases.

### Background Art

In the process of embryogenesis, nerve cells are formed in excess in the initial stage, and then the number thereof is reduced. This process is called the naturally occurring cell death. For example, in lumber region of spinal cord, about 40% of motoneuron are observed to die. This death of motoneuron is observed to progress in agreement with the forming period of neuromuscular synapse junctions. Experimental removal of skeletal muscle prior to innervation of the skeletal muscle, a target tissue, during the embryogenesis causes death of the motoneuron. When taking out and planting limb buds from the other embryo during the process of embryogenesis, only a slight number of motoneuron die. It is therefore assumed that motoneuron scramble for trophic factors derived from the target tissue in a limited amount, and as a result, motoneuron failing to catch such nutritional factors die. Regarding the natural cell death mechanism of motoneuron, the concept that death or alive is dependent on the target tissue is generally accepted.

The nerve growth factor (NGF) is a prototype of trophic factors, and is found to promote of survival of developing sympathetic ganglion nerve cells and some sensory nerve cells. Regarding motoneuron, NGF exerted no effect on the naturally occurring cell death, but rather increases the death of cells caused by axon cutoff in neonatal. Since the 1970s, research efforts have been made for isolation of factors other than NGF considered to participate in peripheral and central nerves. Factors isolated to date include a brain-derived neurotrophic factor (BONF), neurotrophine-3 (NT-3), leukemia inhibiting factor (LIF), and glia-cell-line-derived neurotrophic factor (GDNF), which are found to hinder death of motoneuron in vivo as well as in vitro. Any of them was not isolated from the skeletal muscle, but they are recognized to express in the skeletal muscle. However, selective destruction of mouse gene encoding any of NGF, BDNF, NT-3, LIF or GDNF exerted almost no influence on survival of motoneuron. Effect of a plurality of defective genes is not sufficiently known, the result of knockout of these genes suggests that these factors are not truly motoneuron survival factors, and other molecules remain to be identified in the skeletal muscle.

There is available a supposition that a group of nerve denaturation diseases are brought about by an abnormality of specific neurotrophic factors, and the neurotrophic factors have a hidden potentiality for the therapy of neurodegenerative diseases. A clinical test was carried out on these factors as to patients of amyotrophic lateral sclerosis (ALS), one of the motoneuron diseases. For the time being, however, these factors are determined to be of no effect. It is therefore expected that quite a new factor (or a composite factor group) having potentiality to alleviate ALS or other motoneuron diseases will become available as a therapeutic drug.

The present inventors carried out extensive studies to solve the above-mentioned problems, and successfully isolated a component bringing about a survival effect on motoneuron from chicken skeletal muscle by using a prescribed column chromatographic system (Japanese Unexamined Patent Application Publication No. 2000-287683).

In the study disclosed in the Japanese Unexamined Patent Application Publication No. 2000-287683, the present inventors obtained a physiological activity component exhibiting a survival activity for spinal cord motoneuron. In the initial stage of research, they had anticipated that, like the nutritional factors so far known, protein or polypeptide would be isolated as viability components. The newly isolated activity components were not however protein or polypeptide. More specifically, the present inventors carried out several purifying steps by using a skeletal muscle of a chick embryo sequentially while repeating trials and errors. As a result, they found that physiological activity components showing viability activity for the spinal cord motoneuron were perhaps RNA, a single component containing a part of molecular structure of RNA as a component (for example, RNA derivative, modified or compound) a compound component system containing any of the above, or a mixed component system containing any of the above. However, it is not as yet known whether the actual configuration of the component is a single RNA, an RNA derivative, or a compound component system containing RNA or an RNA derivative as a component (for example, a compound or a mixture), and they could not completely reach a conclusion.

### Disclosure of Invention

The present invention was achieved in view of the above-mentioned circumstances, and has an object to identify essential components of an extracted fraction showing a physiological activity such as a survival activity for neurons, particularly spinal cord motoneuron, determine the structure thereof, and provide a bio-derived component useful for diagnosis, therapy and prevention of neurodegenerative diseases and various other diseases.

To achieve the above-mentioned object, the present inventors further carried out studies on the basis of the findings obtained from the study disclosed in Japanese Unexamined Patent Application Publication No. 2000-287683, and unlike the conventional separating and purifying method disclosed in the Publication, tried a new process comprising the steps of directly extracting RNA from skeletal muscle of a chick embryo, separating an essential component having an activity to maintain and promote survival of spinal cord motoneuron from the thus extracted total RNAs, and identifying the same, and succeeded in this effort.

That is, the present invention provides an RNA exhibiting neuron survival activity, having the following base sequence as specified as sequence number 1 in the sequence table described later. This RNA was named mns-RNA by the present inventors.

(Base sequence of sequence No. 1)
gccgggcgcg guggcgcacg ccuguagucc cagcuacucg ggaggcugag cccgccggau 60
cgcuugagcc caggaguucu gggcugcagu gcgcuaugcc gagcgggcgu ccgcgcuaag 120
gccggcauca auauggugag ccccggggag ccgaggcaca ccagguugcc uaaggagggg 180
ugaaccggaa caggucggaa acggagcagg ucaaaacucc cgugccgguc aguaacggga 240
ucgcgccugu gaauagccac ugcagcguag ccugggcaac auagcgagac ccugucucc 299

The mns-RNA comprising the above-mentioned base sequence No. 1 was confirmed to exhibit a survival activity in vitro on the spinal cord motoneuron.

### Brief Description of the Drawings

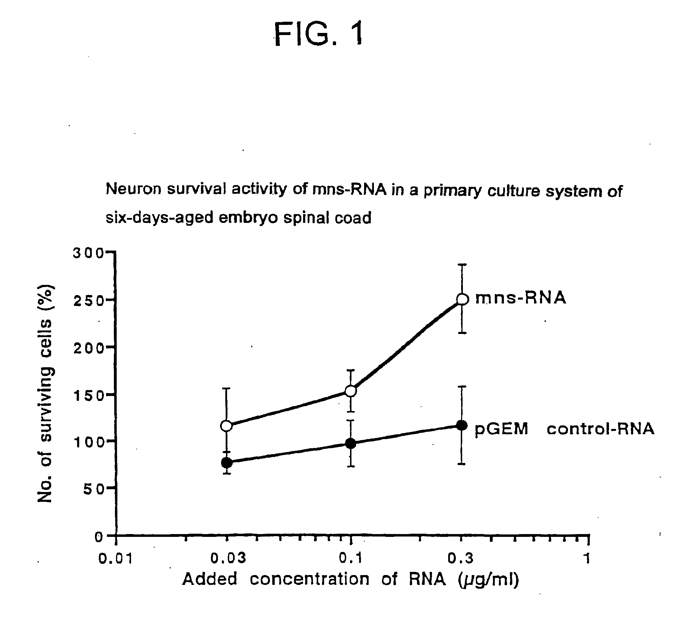

Fig. 1 is a graph illustrating the spinal cord motoneuron survival activity of an RNA having a specific base sequence of the present invention.

### Best Mode for carrying Out the Invention

In order to obtain mns-RNA which is the RNA identified in the present invention, it is desirable to use a six-day-aged or older embryo, natural death of motoneuron beginning to occur at the six-day age, as a raw material chick embryo skeletal muscle, since the above-mentioned RNA is estimated to participate in naturally occurring cell death of motoneuron in the chick embryo and is generated in large quantities during a period in which naturally occurring cell death of motoneuron is active.

RNA of the chick embryo skeletal muscle may be extracted by a known method. For example, the RNA can be extracted by using ISOGEN (commercial name) or ISOGEN-LS commercially available from Nippon Gene Co., Ltd.

About 20,000 to 30,000 kinds of RNA are estimated to be contained in the thus extracted RNA (total RNA). One of the key points of the present invention is therefore how to confirm presence of a specific RNA having the target physiological activity in these kinds of RNA, and how to isolate and identify the target particular RNA of which the presence is confirmed.

First, for confirmation of the target RNA, the method established in the study disclosed in the above-mentioned Publication No. 2000-287683 is used. Confirmation is accomplished from the fact that RNA exhibits a survival activity in a bioassay system using cultured neurons, the survival activity thereof becomes invalid by an RNA degradation enzyme, and that the survival activity can be intensified under the effect of an RNA degradation enzyme activity inhibitor (RNase inhibitor).

The extracted total RNA may be cloned as it is by a cDNA library preparing technique for identification. In the process of study of the present invention, the target RNA was found to be contained in the total RNA in a content of only slightly lower than 1 %. Isolation of the target RNA was therefore tried through chromatography.

More specifically, it was tried to elute the total RNA by means of a DEAE cephalose column while gradually changing the concentration of saline. That is, the concentration of salt in the elute was increased stepwise from 0 M to 0.1 M, 0.2 M, 0.3 M, 0.5 M, 1.0 M and then to 5.0 M, and for each of the resultant fractions, a test was carried out in the above-mentioned bioassay system. A strong activity was detected in the 5 M saline eluted fraction, and RNA was contained in a percentage of only 0.07 % of the total RNA. Concentration of the neuron survival activity in this fraction was observed.

While the survival activity is slightly observed also in the 1 M saline elute fraction, most is contained in the 5 M saline elute fraction. In general, in a saline having a high concentration over 1 M, an RNA of a very low molecular weight is eluted. In view of this fact, elution of the RNA of the present invention in a saline having a high concentration as 5 M was therefore an unexpected result.

Then, using the above-mentioned 5 M saline elute fraction, a cDNA library is prepared with RNA contained in this elute fraction as the template, and the base sequence of each clone of the thus prepared cDNA library is determined. It is thus possible to identify the target RNA having a survival activity relative to motoneuron by amplifying the template RNA through in vitro transcription by using the individual clones of the cDNA library, and screening the survival activity by means of a bioassay system using cultured neuron.

### Examples of experiment

The present invention will now be described further in detail by means of examples of actual experiment.

### A. Confirmation of survival activity by total RNA

### (1) Experimental materials

Fertilized eggs of white leghorn were purchased in an ordinary market and incubated at 37 C for six or 19 days in a humidified incubator. Embryos resulting from incubation (corresponding to stage 29 or stage 45, respectively, of Hamburger Hamilton) were used.

### (2) Purification of RNA

ISOGEN (made by Nippon Gene Co., Ltd.) in an amount of 100 mL was poured into four 50 mL plastic tubes by 25 mL each; a chicken skeletal muscle was added by 5 g each; and the mixture was homogenized by using a polytron homogenizer. After homogenization, the tubes were held at room temperature for five minutes; chloroform was added by 5 mL each; the tubes were vigorously shook for 15 seconds; and held at room temperature for a few minutes. Then, centrifugal separation was performed at 12,000 rpm and 4 C for 15 minutes, and the supernatant was transferred to four new plastic tubes. Isopropanol was added by 12.5 mL each to supernatant of each plastic tube, and the tubes were held at room temperature for five to ten minutes. Centrifugal separation was performed again for each plastic tube at 12,000 rpm and 4 C for 15 minutes. Thus obtained precipitate was washed by 70 % ethanol solution. Precipitate after washing was slightly dried, and dissolved into sterilized water by 1 mL each. The resultant product was stored at -80 C as a total RNA and used for experiments described later.

### (3) Preparation of bioassay system

A primary culture system of spinal cord neuron was obtained from a six-day-aged embryo of the above-mentioned fertilized eggs of white leghorn to investigate neuron survival activity resulting from the total RNA. The six-day-aged embryo was used because naturally occurring cell death of motoneuron begins in this stage.

A six-days-aged embryo was placed in an ice-cooled Dulbecco's modified Eagle's medium (DMEM, gibco BRL) to which antibiotics [penicillin (10 units/mL, Meiji Pharmaceutical) and streptomycin (50 µg/mL, Meiji Pharmaceutical)], and 10 % heat inactivated fetal bovine serum (FBS) (Mitsubishi Chemical Industries Ltd.) were added, and the spinal cord was picked out by using scissors while watching through a stereoscopic microscope in this mixture. The spinal cord thus picked out was incubated at 37 C for 15 minutes by using a stirring vessel (about 70 cycles/minute) in 10 mL of 0.25 % trypsin-containing Ca²⁺ and Mg²⁺ free phosphate buffered saline. The reaction of trypsin was discontinued by adding 2 mL heat inactivated horse serum. After centrifugally separating it at 1,000 rpm for five minutes, the supernatant was removed, and 6 mL DMEM/TIP [i.e., DMEM to which transferrin (5 µg/mL, Sigma), insulin (5 µg/mL, Collaborative Res.), progesterone (0.2 µM, Sigma) and the antibiotics as described above] were added to the precipitate. The mixture was stirred by repeating a cycle comprising suction and discharge by means of a plastic chip 20 times to separate cells from each other. The resultant spinal cord neuron suspension was filtered through a nylon mesh (#150). Then, the filtered suspension was diluted for culture by using a DMEM/TIP medium so as to give 1 x 10⁵ cells/mL. The well (culture plate) for culture of tissue (15 mm dia., Sumitomo) was precoated with polyethyleneimine for at least four hours. The precoated plate was rinsed twice with sterilized distilled water, and a DMEM containing antibiotics was added. The separated spinal cord neuron was plated at a concentration of 1 mL/well. The concentration corresponded to 200 cells/mm².

### (4) Confirmation of survival activity

The total RNA and skeletal muscle rough extract in a slight amount to an extent not exhibiting survival activity were added to the well onto which the cultured cells were sprinkled and a bioassay was carried out. The total RNA was added in an amount of 0.1 µg, 1.0 µg, 10 µg or 100 µg per well, respectively.

Bioassays were carried out also for each total RNA which was treated by RNase and RNase inhibitor, respectively. More specifically, the total RNA was treated by RNase (Boheringer Mannheim, 50 units/mL, 37 C for three hours) or treated by RNase inhibitor (Promega, 4 units/3 µL, room temperature for three hours), and an equal amount of phenol/chloroform/- isoamyl alcohol (25 : 24 : 1) was added and shook. Centrifugal separation was conducted at 15,000 rpm for five minutes, and precipitation of supernatant was caused with ethanol. The resultant precipitate was centrifugally separated at 15,000 rpm and 4 C for 20 minutes, and the precipitate dissolved in sterilized water was added to the well onto which cultured cells were sprinkled in the same manner as described above.

Then, after incubation at 37 C for two days in a humidifier containing 5 % CO₂ and 95 % air, the number of nerve cells surviving in a prescribed area (895 µm x 1340 µm) of each well was counted by means of a phase-contrast microscope (manufactured by Nikon Corp.).

In this bioassay system, the number of surviving nerve cells was larger in the total RNA than in the control, and the number of surviving cells was slightly larger in the total RNA which was treated by RNase inhibitor than in the total RNA not treated. The survival activity of nerve cells was recognized in the total RNA from this result.

### B. Preparation of cDNA library and screening

### (1) Chromatography

A chromatography was carried out by passing the above-mentioned total RNA through a DEAE cephalose column. More specifically, total RNA in an amount of 5 mg extracted from a 18-days-aged embryo chicken skeletal muscle by using ISOGEN was dissolved into 10 mM Tris-HCI in an amount of 0.25 mL, and the total RNA was separated by increasing the concentration of NaCl stepwisely from 0 M to 0.1 M, 0.2 M, 0.3 M, 0.5 M, 1.0 M and then to 5.0 M by using 1 mL DEAE cephalose fast flow column (Amersham Pharmacia Biotech).

An assay was carried out by means of the above-mentioned bioassay system for each of the resultant fractions. For the 5 M NaCl eluted fraction, the RNA content is represented by 0.07 % of the total RNA. A strong activity was detected, and neuron survival activity was concentrically observed in this fraction.

### (2) Preparation of cDNA library

By using the above-mentioned 5 M NaCl eluted fraction, a cDNA library using the RNA contained in the fraction as a template was prepared. RNA in the 5 M NaCl eluted fraction was first dissolved in sterilized water in an amount of 5 µL, and a cDNA was synthesized by using a cDNA synthesizing kit (Takara). In this case, a 6 mer random primer (Takara) was used as a primer. Synthesis of a first strand was gradually conducted under conditions including the reaction temperature and the reaction time of 42 C for ten minutes, 47 C for ten minutes and then 52 C for ten minutes. An equal amount of phenol/chloroform/isoamyl alcohol (25 : 24 : 1) solution was added to the reaction solution after cDNA synthesis. The mixture was shaken, and centrifugal separation was applied at 15,000 rpm for five minutes. After passing the supernatant through a microspin column (Amersham Pharmacia Bioteck), precipitation was caused with ethanol.

The resultant cDNA obtained as precipitate was dissolved in 4.0 µL sterilized water, and mixed with an EcoRI adapter (100 pmol/0.5 µL). A ligation solution I of a DNA ligation kit (Takara) in an amount of 4.5 µL was added to cause a reaction, and an adapter was attached to cDNA. In this reaction, the reaction temperature and the reaction time were divided into three stages of 4 C for 20 minutes, 12 C for 30 minutes and 16 C for 30 minutes.

Then, phenol/chloroform/isoamyl alcohol was added to the reaction solution, and the mixture was shaken. Then, the mixture was centrifugally separated at 15,000 rpm for five minutes, and precipitation was caused with ethanol. The precipitate was dissolved into 40 µL sterilized water, and 2.5 µL EcoRI and 4 µL EcoRI buffer were added. The mixture was caused to react at 37 C for an hour, and cut off with EcoRI. Then, an equal amount of phenol/chloroform/isoamyl alcohol was added to the reaction solution, and the mixture was shaken. Centrifugal separation was conducted at 15,000 rpm for five minutes, and the supernatant was passed through a microspin column (Amersham Pharmacia Bioteck) to cause ethanol precipitation. The half amount of the reaction product and 0.5 µg of λgt10 were mixed together and caused precipitation by ethanol. The precipitate was dissolved in 3 µL sterilized water, and dried to reach 1.7 µL. The ligation solution I in an amount of 1.7 µL of a DNA ligation kit (Takara) was added to carry out ligation.

In vitro packaging into a phage was accomplished by use of a GIGA PACK Packaging extract (Stratagene), and a library was prepared by causing infection to Escherichia coli (C600). The insertion portion of about 90 cDNA clones contained in this library was amplified through a polymerase chain reaction (PCR) by using the primer at the portion contained in λgt10. A sequence reaction was caused by use of a Dye terminator Cycle sequencing Ready Reaction kit (Perkin-Elmer), and a base sequence was determined by using an ABI sequencer for each of about 90 cDNA clones.

### (3) Screening of survival activity

After determination of the base sequence, the RNA corresponding to the original template was amplified by the in vitro transcription technique from each clone of the cDNA library to assay a neuron survival activity with the above-mentioned bioassay system. More specifically, skeletal muscle rough extract in a slight amount to an extent not exhibiting a survival activity relative to the RNA synthesized by the in vitro transcription technique was added onto the well having sprinkled cultured cells. The RNA synthesized by the in vitro transcription technique was added in an amount of 0.03 µg, 0.1 µg or 0.3 µg per well.

Then, after incubation at 37 C for two days in a humidifier containing 5 % carbon dioxide gas and 95 % air, the number of nerve cells surviving in a prescribed area (895 µm x 1340 µm) of each well was counted by use of a phase-contrast microscope (manufactured by Nikon Corp).

As a result, as shown in Fig. 1, the RNA derived from a clone was specified as an RNA exhibiting a strong spinal cord motoneuron survival activity in a manner depending upon concentration. This RNA has the base sequence of the above-mentioned sequence No.1, and was named mns-RNA.

### Industrial Applicability

As described above, mns-RNA comprising the base sequence of the sequence No.1 shows a survival activity in vitro relative to the spinal cord motoneuron. It is therefore considered that mns-RNA has high probably an important physiological activity also in vivo relative to the spinal cord motoneuron, and is expected to provide a high applicability to therapy, prevention and diagnosis of amyotrophic lateral sclerosis (ALS) which is a neurodegenerative disease of the spinal cord motoneuron.

The mns-RNA having the base sequence of sequence No.1 of the present invention may exhibit an important physiological activity also for nerve cells other than spinal cord motoneuron, particularly, spinal cord neuron (for example, interneuron) other than spinal cord motoneuron, motoneuron of the portions other than the spinal cord, and other neurons. It may therefore be applicable for diagnosis, therapy and prevention of diseases of motoneurons other than ALS in which the nerve cells participate in some form or other, for example, spinal progressive muscular atrophy and Werdnig-Hoffman disease. It may also be applicable for therapy and prevention of nerve denaturation diseases such as Parkinson disease, Hantington disease and Alzheimer disease.

It may therefore be applicable as a neuron survival promoting drug or antimotor neuron disease drug containing mns-RNA of the base sequence of sequence No. 1 of the present invention as an essential component.

In order to use the mns-RNA of the present invention as a diagnostic drug of motoneuron diseases, it would be necessary to extract DNA from blood or mucous membrane sampled from patients of motoneuron disease, on the assumption of confirmation of the presence in the human normal muscle, investigate the base sequence of genome DNA coding the mns-RNA, and compare it with the sequence of this RNA. If there is a defective portion in the base sequence or substitution of the base, this can be estimated as an RNA relating to a motoneuron disease. It is therefore possible to diagnose the possibility of a motoneuron disease. As therapeutic drugs, a method of transplanting cells incorporating DNA encoding this RNA or planting a capsule containing the same into tissue.

## Claims

1. An RNA having a base sequence of sequence No.1 and exhibiting a neuron survival activity.
